# EUROPÄISCHE PATENTANMELDUNG

(11) **EP 1 308 167 A1**
(43) Veröffentlichungstag der Anmeldung: **07.05.2003**
(21) Anmeldenummer: 01126343.1
(22) Anmeldetag: 06.11.2001
(51) Int. Cl.: A61K 39/385, A61K 39/00, C12N 5/10, C12N 15/86, C07K 14/705

(54) **Antigenpräsentierende Vesikel**

(71) Anmelder: Pickl, Winfried, Ao. Univ. Prof. Dr., 1080 Wien (AT)
(72) Erfinder: Die Erfindernennung liegt noch nicht vor
(74) Vertreter: Rosenich, Paul

(57) **Zusammenfassung**

Die vorliegende Erfindung betrifft subzelluläre antigenpräsentierende Vesikel (SAV), welche dadurch gekennzeichnet sind, dass sie von transfizierten Zellen produziert werden und die relevanten Moleküle für Erst- und gegebenenfalls Zweitsignale zur antigenspezifischen Aktivierung von T-Lymphozyten bereitzustellen vermögen. Des weiteren betrifft die vorliegende Erfindung SAV, welche dadurch gekennzeichnet sind, dass sie ausschließlich Erstsignale oder aber Erstsignale in Kombination mit sogenannten inhibitorischen Zweitsignalen zur antigenspezifischen Inhibition von T-Lymphozyten bereitzustellen vermögen.

Ferner betrifft die Erfindung ein Verfahren zur Herstellung dieser Vesikel und diese Vesikel enthaltende Vakzinen sowie weitere Anwendungsmöglichkeiten.

SEQUENCE LISTING

<110>  Pickl, Winfried

<120>  Antigenpräsentierende Vesikel

<130>  A49EP

<140>  EP 01126343.1
<141>  2001-11-06

<160>  18

<170>  PatentIn version 3.1

<210>  1
<211>  35
<212>  DNA
<213>  Artificial

<220>
<221>  misc_feature
<222>  (1)..(35)
<223>  forward primer

<400>  1
ggaattcgct agcccaggtc cagctgcagc agtct                                35

<210>  2
<211>  36
<212>  DNA
<213>  Artificial

<220>
<221>  misc_feature

<222>  (1)..(36)

<223>  reverse primer


<400>  2
gggggatccg gtgaccgtgg tgccttggcc ccagta                                36

<210>  3

<211>  38

<212>  DNA

<213>  Artificial


<220>

<221>  misc_feature

<222>  (1)..(38)

<223>  forward primer


<400>  3
ggaattcgag ctcccaaatt gttctcaccc agtctcca                              38

<210>  4

<211>  39

<212>  DNA

<213>  Artificial


<220>

<221>  misc_feature

<222>  (1)..(39)

<223>  reverse primer


<400>  4
gggatcccca ccgccccggt ttatttccaa ctttgtccc                             39

<210>  5

<211>  33

<212>  DNA

<213>  Artificial

<220>

<221> misc_feature

<222> (1)..(33)

<223> oligonucleotide sequence (forward) used for making a molecule sec
tion coding for a CD80 ectodomain

<400> 5
cgcgggggat cccgttatcc acgtgaccaa gga 33

<210> 6

<211> 31

<212> DNA

<213> Artificial

<220>

<221> misc_feature

<222> (1)..(31)

<223> oligonucleotide sequence (reverse) used for making a molecule sec
tion coding for a CD80 ectodomain

<400> 6
gcgccgctag cggatgggag caggttatca g 31

<210> 7

<211> 33

<212> DNA

<213> Artificial

<220>

<221> misc_feature

<222> (1)..(33)

<223> oligonucleotide sequence (forward) used for making a molecule sec
tion coding for a CD86 ectodomain

<400> 7
cgcgggggat cccgctcctc tgaagattca agc 33

<210> 8

<211> 32

Seite 3

<212>  DNA

<213>  Artificial

<220>

<221>  misc_feature

<222>  (1)..(32)

<223>  oligonucleotide sequence (reverse) used for making a molecule sec
       tion coding for a CD86 ectodomain

<400>  8
gcgcccgcta gcgtggtctg ggggaggctg ag                                    32

<210>  9

<211>  33

<212>  DNA

<213>  Artificial

<220>

<221>  misc_feature

<222>  (1)..(33)

<223>  oligonucleotide sequence (forward) used for making a molecule sec
       tion coding for a CD40 ectodomain

<400>  9
cgcgggggat cccgaaccac ccactgcatg cag                                   33

<210>  10

<211>  32

<212>  DNA

<213>  Artificial

<220>

<221>  misc_feature

<222>  (1)..(32)

<223>  oligonucleotide sequence (reverse) used for making a molecule sec
       tion coding for a CD40 ectodomain

<400>  10
gcgcccgcta gccaccaggg ctctcagccg at                                    32

<210>  11

<211>  33

<212>  DNA

<213>  Artificial

<220>

<221>  misc_feature

<222>  (1)..(33)

<223>  oligonucleotide sequence (forward) for making a CD16-GPI anchor s
       equence

<400>  11
cgcggggcta gcttggcagt gtcaaccatc tca                                   33

<210>  12

<211>  37

<212>  DNA

<213>  Artificial

<220>

<221>  misc_feature

<222>  (1)..(37)

<223>  oligonucleotide sequence (reverse) for making a CD16-GPI anchor s
       equence

<400>  12
gcgcccgcgg cgctttaaat gtttctcttc acagaga                               37

<210>  13

<211>  33

<212>  DNA

<213>  Artificial

<220>

<221>  misc_feature

<222>  (1)..(33)

<223>  oligonucleotide sequence (forward) for making a CD59-GPI anchor s
       equence

<400>  13
ggattcgcta gccttgaaaa tggtgggaca tcc                                    33

<210>  14

<211>  38

<212>  DNA

<213>  Artificial

<220>

<221>  misc_feature

<222>  (1)..(38)

<223>  oligonucleotide sequence (reverse) for making a CD59-GPI anchor s
       equence

<400>  14
cccgcggcgg ccgctttagg gatgaaggct ccaggctg                               38

<210>  15

<211>  30

<212>  DNA

<213>  Artificial

<220>

<221>  misc_feature

<222>  (1)..(30)

<223>  oligonucleotide sequence (forward) used for modification of the H
       LA-A*02011 gene

<400>  15
cgcgggggat ccctgaccca gacctgggcg                                        30

<210>  16

<211>  31

<212>  DNA

<213>  Artificial

<220>

<221>  misc_feature

<222>  (1)..(31)

<223>  oligonucleotide sequence (reverse) used for modification of the H
       LA-A*02011 gene

<400>  16
cgcgggggat cccctgaccc agacctgggc a                                    31

<210>  17

<211>  38

<212>  DNA

<213>  Artificial

<220>

<221>  misc_feature

<222>  (1)..(38)

<223>  gag-specific primer

<400>  17
cgcggggcgg ccgctttagt catctagggt caggaggg                             38

<210>  18

<211>  19

<212>  DNA

<213>  Artificial

<220>

<221>  misc_feature

<222>  (1)..(19)

<223>  forward primer

<400>  18
cgactcacta tagggagac                                                  19

## Beschreibung

### TECHNISCHES GEBIET

Die vorliegende Erfindung, die überwiegend in den Bereich der Immunologie fällt, bezieht sich auf antigenpräsentierende Membranvesikel, insbesondere auf subzelluläre antigenpräsentierende Vesikel (SAV), welche von transfizierten Zellen produziert werden und relevante Moleküle für Erstsignale, gegebenenfalls auch für Zweitsignale, zur antigenspezifischen Aktivierung oder aber zur antigenspezifischen Anergisierung von T-Lymphozyten bereitzustellen vermögen. Ferner betrifft die Erfindung ein Verfahren zur Herstellung dieser Vesikel und diese Vesikel enthaltende Vakzinen sowie weitere Anwendungsmöglichkeiten.

### STAND DER TECHNIK

Es ist bekannt, dass dendritische Zellen, welche von Monozyten oder CD34⁺ Stammzellen abstammen, extrem potente antigenpräsentierende Zellen (APZ) sind, welche imstande sind, tumorassoziierte und virale Antigene in vivo zu präsentieren. Solche Zellen sind weiters dazu imstande, spezifische Immunität gegen bestimmte Arten von Krebs bzw. Infektionskrankheiten zu induzieren. Zu diesem Zweck exprimieren sie bestimmte Oberflächenmoleküle, welche zur Aktivierung von naïven T-Zellen führen können.
Der Nachteil einer therapeutischen Anwendung der oben beschriebenen Zellen liegt darin, dass man sie in großer Anzahl und unter GMP Bedingungen vom jeweiligen, histokompatiblen Patienten ex vivo herstellen muss.

Bereits bekannt sind Verfahren, bei denen durch Transfektion (Latouche and Sadelain, 2000; Sprent et al., 1997) mit einzelnen HLA-Molekülen und T-Zell-kostimulatorischen Molekülen künstliche APZ erzeugt werden konnten. Koexpression bzw. -transfektion von Adhäsionsmolekülen, wie z.B. ICAM-1, erhöht das Effektorzellen induzierende Potenzial der APZ (Camacho et al., 2001).

### BESCHREIBUNG DER ERFINDUNG

Der gegenständlichen Erfindung liegt die Aufgabe zugrunde, ein Mittel bereitzustellen, das von Patientenzellen unabhängig die antigenpräsentierende Funktion von aus Blutzellen oder CD34⁺ Stammzellen hergestellten dendritischen Zellen ausübt.

Unter HLA-Molekülen versteht der Immunologe jene Moleküle, welche am menschlichen Chromosom 6 codiert sind und welche im Falle von Klasse I Molekülen aus einer polymorphen α-Kette und dem monomorphen β₂-Microglobulin bestehen, im Falle von Klasse II Molekülen jedoch aus einer polymorphen α- und einer polymorphen β-Kette. Die Aufgabe dieser HLA-Moleküle ist es, Protein-Fragmente in Form von Oligopeptiden zu binden und den Effektorzellen des Immunsystems, vor allem den T-Zellen, darzubieten.
Der Ausdruck "kostimulatorisches Molekül" umfasst in diesem Zusammenhang jedes Molekül, das im Beisein eines antigenspezifischen T-Zellstimulus diesen verstärken bzw. seine Wirkung über den erforderlichen Schwellenwert hinaus anheben kann, sodass die naive T-Zelle aktiviert wird. Ein solches kostimulatorisches Molekül kann z.B. ein membranständiger Rezeptor sein. Im Falle einer APZ sind insbesondere die Rezeptoren CD80, CD86 und CD58, sowohl alleine als auch in beliebiger Kombination miteinander, für die vorliegende Erfindung geeignet.

Mit dem Begriff "Adhäsionsmolekül" werden Moleküle bezeichnet, die einen physischen Kontakt zwischen zwei Zellen bewirken oder verstärken und damit weitere Interaktionen zwischen diesen Zellen begünstigen. Es handelt sich hierbei großteils um zellständige Rezeptormoleküle. Bei der Interaktion einer APZ mit einer T-Zelle sind unterschiedliche Adhäsionsmoleküle auf der APZ beteiligt, so z.B. CD54 (ICAM-1) oder DC-SIGN (CD209).

Der Ausdruck "Antigene Peptide" umfasst vor allem jene Fragmente von Fremd- oder Tumorproteinen, welche im Zytoplasma von humanen Zellen durch die proteolytische Wirkung von sogenannten Proteasomkomplexen bereitgestellt werden und welche über Vermittlung von Peptidtransportern in das Endoplasmatische Retikulum eingeschleust werden. Aufgrund ihrer primären Aminosäuresequenz wirken nur jene Fremd- oder Tumorpeptide als Antigene, welche von der sogenannten antigenbindenden Grube von HLA Molekülen gebunden werden. In diese Gruppe fallen z.B. virale Antigene, tumorassoziierte Antigene, aber auch Allergene und Autoantigene.

Kombinationen von relevanten Vertretern dieser vier Molekülgruppen auf ein und derselben Zelle führen zur Entstehung einer APZ mit spezifischer T-Zell-aktivierender oder gegebenenfalls T-Zell-inhibierender Fähigkeit. Mit der vorliegenden Erfindung wird die antigenpräsentierende Einheit als zellfreies System adaptiert, wobei die HLA-Moleküle, die kostimulatorischen Moleküle, die Adhäsionsmoleküle und die antigenen Peptide in konzentrierter und konzertierter Form wirken und je nach Erfordernis variiert werden können.

Erfindungsgemäß wird dies durch Bereitstellung von SAV errreicht, welche die Moleküle zur Antigenpräsentation und T-Zellaktivierung bzw. T-Zellinhibierung (Anergisierung) an ihre Oberfläche gebunden tragen. Die SAV enthalten somit in Analogie zu einer antigenpräsentierenden dendritischen Zelle die wesentlichen Elemente, welche zur Formierung der sogenannten immunologischen Synapse zwischen APZ und T-Zelle vonnöten sind.

Dem Fachmann sind natürliche Arten von Vesikelbildung durch Zellen bekannt, so z.B. die Formierung von Exosomen (Johnstone and Ahn, 1990). Es ist weiters bekannt, dass auch natürliche dendritische Zellen subzelluläre Vesikel abgeben, welche Oberflächenmoleküle der dendritischen Zelle enthalten (Thery et al., 2001; Zitvogel et al., 1998). Dem gegenüber handelt es sich bei der vorliegenden Erfindung um Vesikel, welche sich in ihrer Aktivität zwar eng an die natürliche Wirkungsweise anlehnen, die jedoch mittels gentechnischer Methoden erzeugt werden und vielfältig modifizierbar sind.

Auch bei der Produktion von Hüllenviren aus infizierten Zellen werden Plasmamembranabschnitte in Form von Vesikeln abgegeben. Dafür sind die sogenannten Coreproteine von Retroviren, z.B. von Moloney murine leukemia virus (Moloney MLV), zuständig. Diese Coreproteine werden in der Plasmamembran in sogenannten Lipidrafts (das sind cholesterin-, glycosphingolipid- und gangliosidreiche Membranbereiche) konzentriert Ebenfalls konzentrieren sich die Envelopeproteine (Hüllenproteine) phylogenetisch unterschiedlicher RNA- und DNA-Viren in solchen Bereichen, wie auch die Virusabschnürung an sich in diesen Bereichen vonstatten geht (Pickl et al., 2001).

Erfindungsgemäß werden virale Coresequenzen zur induzierten und somit verstärkten Vesikelbildung eingesetzt. Unter "viralen Coresequenzen" versteht man virale, insbesondere retrovirale, Coreproteine oder Teile von Coreproteinen, wie beispielsweise die "group specific antigens" (GAG) von Moloney MLV und HIV oder das M1 Protein von Influenzavirus.

Wie gezeigt werden konnte, dürfte die posttranslationelle Lipidmodifikation von entsprechenden viralen oder aber auch zelleigenen Proteinen für deren Anreicherung in Lipidrafts und somit in Hüllen von Hüllenviren verantwortlich sein (Pickl et al., 2001). "Posttranslationelle Lipidmodifikation" bezeichnet im allgemeinen die kovalente Anfügung einer oder mehrerer Fettsäuren, zumeist ungesättigter Fettsäuren, an das zu modifizierende Protein im Endoplasmatischen Reticulum, was zur bevorzugten Verankerung des modifizierten Proteins in den Lipidrafts der Plasmamembran führt (Melkonian et al., 1999; Pessin and Glaser, 1980). Erfindungsgemäß von Bedeutung sind insbesondere Myristoylierung, Palmitoylierung und Glykosylphosphatidylinositol (GPI) -Verankerung.

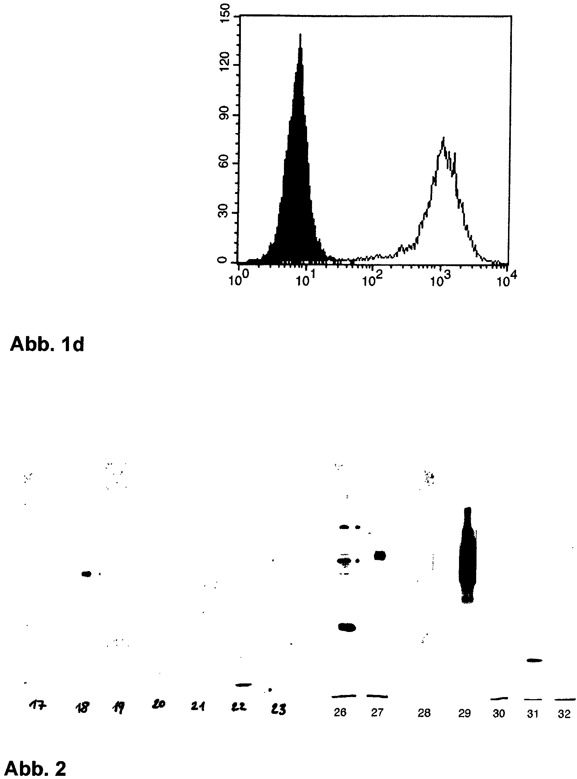

In der vorliegenden Erfindung werden u.a. auch solche modifizierten Proteine eingesetzt. Dabei handelt es sich vorzugsweise um die Ektodomänen von humanem CD80 (B7.1), CD86 (B7.2), CD54 (ICAM-1), CD58 (LFA-3) sowie von HLA-Klasse I und II Molekülen jeglichen Allels. Zusätzlich enthalten die erfindungsgemässen SAV nachweislich die von der 293T Zelllinie konstitutiv exprimierten humanen GPI-verankerten Moleküle CD55 und CD59 *(Abb. 2*), welche inhibierend auf die Komplement-Kaskade wirken und daher den raschen Abbau der Vesikel im Körper verzögern können (Marschang et al., 1995; Saifuddin et al., 1994).

Vorzugsweise werden die mit Hilfe des pEAK12 Vektors in die 293T Zellen rekombinant eingebrachten Moleküle bzw. Gene überexprimiert. Erreicht wird dies durch Verwendung des EF1α Promoters und anderer dem Fachmann bekannter Mechanismen, wie z.B. der intrazellulären Plasmidamplifikation durch einen SV40 "origin of replication" und der episomalen Stabilität, welche durch einen "EBV origin of replication" vermittelt werden. Das dürfte ebenfalls zur Anreicherung der Genprodukte in Lipidrafts beitragen. Dieser Umstand scheint insbesondere für solche Moleküle relevant zu sein, welche im Rahmen der vorliegenden Erfindung in nichtmodifizierter Form exprimiert wurden (z.B. Typ II Membranproteine wie DC-SIGN (CD209)).

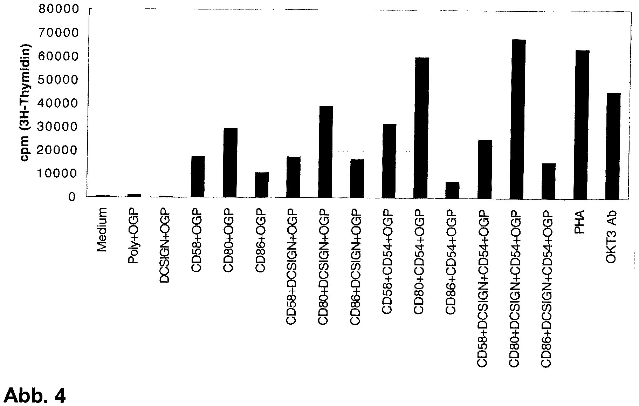

Die erfindungsgemäßen SAV ermöglichen es, Signale der Antigenpräsentation aufT-Zellen zu übertragen, wobei der Komplex HLA-Molekül/antigenes Peptid ganz nach Bedarf patienten- und krankheitsspezifisch abgestimmt werden kann. Die Stärke der T-Zellaktivierung ist dabei abhängig von den auf den erfindungsgemässen SAV gegebenenfalls zusätzlich anwesenden kostimulatorischen Molekülen und Adhäsionsmolekülen. (*Abb. 4*). Das bedeutet, dass unter Weglassung der kostimulatorischen Moleküle auch SAV mit einer sogenannten anergisierenden, z.B. T-Zell-inhibierenden, Wirkung hergestellt werden können. Auch solche SAV sind von der vorliegenden Erfindung mitumfasst. Sie können beispielsweise selektiv auf verschiedene Allergene oder Autoantigene abgestimmt und zur Bekämpfung von Allergien oder Autoimmunkrankheiten therapeutisch eingesetzt werden.

Bei den SAV der vorliegenden Erfindung handelt es sich um ein zellfreies, nichtreplikatives System, welches die Vorteile von synthetischen immunogenen Peptiden oder Bindungsreagenzien und jene von Liposomenvesikel-Technologien vereint. Durch die künstlich induzierte Abschnürung von Plasmamembranvesikeln aus für diesen Zweck geeigneten lebenden, vorzugsweise humanen, Zellen gelingt es, die - durch die Koevolution von viralen und (Säugetier-)Biosystemen auf natürlichem Wege optimierten, physikalisch-biologischen Eigenschaften dieser Vesikel auszunutzen, insbesondere was die Interaktion von Plasmazellmembranen unterschiedlicher Zellen miteinander betrifft.

Die SAV gemäss vorliegender Erfindung erlauben vielfältige Anwendungsmöglichkeiten. Sie eignen sich beispielsweise:
- als Therapeutikum gegen verschiedene Tumorarten;
- als Mittel, um gegen unterschiedliche Antigene, auf die das Immunsystem normalerweise nicht oder nur unzureichend reagiert, eine Immunantwort hervorzurufen;
- als Werkzeug für Screenings von Tumorzellen;
- in abgewandelter Form als Anergisierungsreagenz zur Abschaltung von bereits stattfindenden Immunreaktionen;
- als nützliches Werkzeug zur Analyse neuer kostimulatorischer Moleküle, Adhäsionsmoleküle und anderer Moleküle;
- zur konzentrierten Verabreichung von Botenstoffen und/oder Wachstumsfaktoren, welche im Lumen der SAV angereichert werden können;
- als Plattform für die Herstellung von multivalenten Bindungsreagenzien, wie sie für die antigenspezifische Charakterisierung von Säugetierzellen, insbesondere humanen Immunzellen, verwendet werden.

Der Vorteil von SAV gegenüber antigenpräsentierenden Exosomen liegt in der Tatsache, dass SAV direkt aus der Plasmazellmembran hervorgehen und deren molekulares makeup wie oben beschrieben frei variiert werden kann. Dies ist im Falle von Exosomen nicht möglich. Da Exosomen aus sogenannten multivesikular bodies (MVB) hervorgehen, ist eine Modifikation ihrer molekularen Zusammensetzung nicht mit den oben beschriebenen Methoden möglich. Zudem ist ihr Einsatz auch abhängig vom Histokompatibilitätstypus des Zellspenders.

### BESCHREIBUNG DER ABBILDUNGEN:

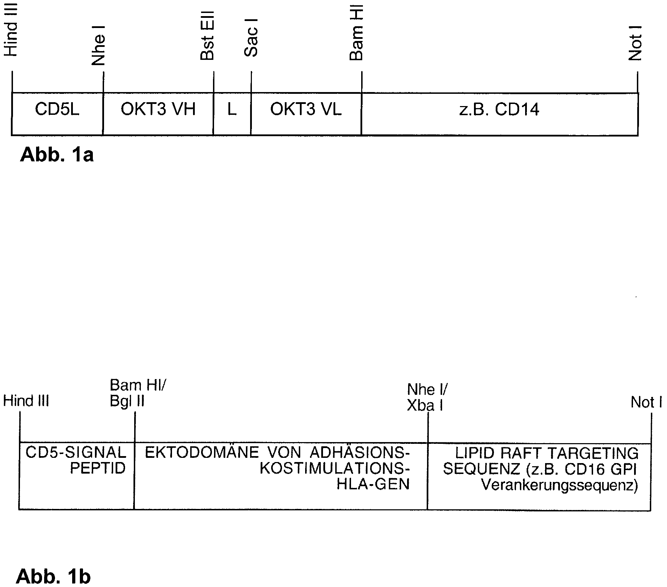

**Abb. 1a:** Schematische Darstellung der Konstruktion der CD5L::CD3-scFᵥ::IgG₁. bzw. CD5L::CD3-scFᵥ::CD14 Expressionskassette (Ausschnitt aus dem Vektor pEAK 12). Die Abbildung zeigt die einzelnen Genabschnitte und die verwendeten RE-Schnittstellen welche zur Klonierung der Einzelabschnitte herangezogen wurden. Gezeigt ist nur das entsprechende Insert, wie es in dem Expressionsvektor pEAK12 zur Verwendung kommt.
**Abb. 1b:** Schematische Darstellung der Expressionskassette zur Klonierung von GPImodifizierten Typ I Transmembranproteinen (Ausschnitt aus dem Vektor pEAK 12). Die Expressionkassette dient der Modifikation von Zelloberflächenrezeptoren, um deren Konzentration in Lipidrafts zu erhöhen. Die Abbildung zeigt nur das entsprechende Insert, wie es in dem Expressionsvektor pEAK12 zur Verwendung kommt.

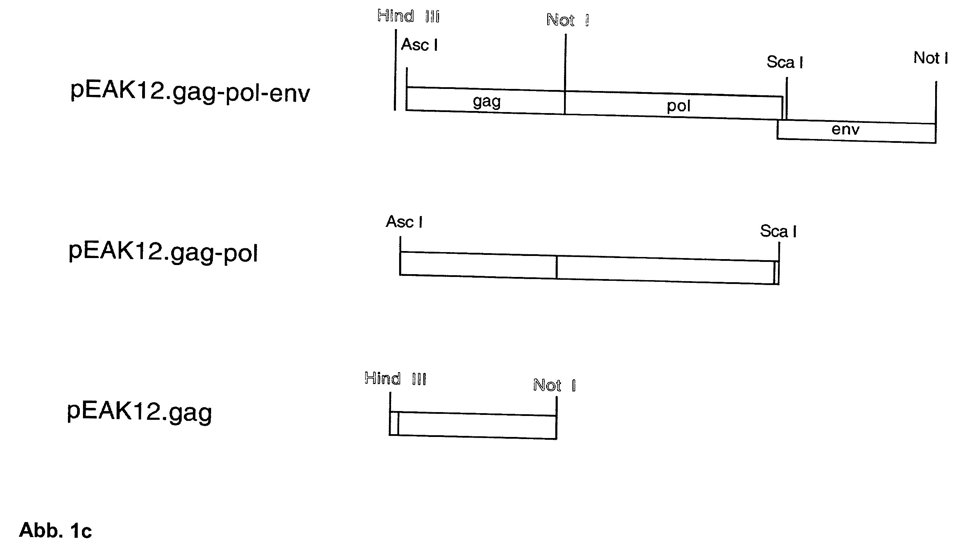

**Abb. 1c:** Schematische Darstellung der Expressionskassette für OGP/OG Konstrukte. Die für die Herstellung der GAG-POL bzw. GAG exprimierenden Versionen von pEAK12 verwendeten RE-Schnittstellen im MoMLV Genom, welche für die Klonierung der Genabschnitte in die multiple cloning site von pEAK12 von Bedeutung sind, werden entsprechend angezeigt. Die Abbildung zeigt nur die entsprechenden Inserts.
**Abb. 1d:** Analyse der stabilen Transfektante mittels Durchfluß-Cytometrie. Die Abbildung lässt die stabile Expression des CD5L::CD3scFv::CD14 Transgens in 293T Zellen (Klon #19) erkennen. Das Bild zeigt ein sogenanntes overlay Histogramm einer flowcytometrischen Untersuchung, wobei sich die Negativkontrollfärbung am linken Bildrand, die Färbung des Transgens am rechten Bildrand als nahezu idealverteilte Kurven darstellen.
**Abb. 2:** Ergebnisse eines Western Blots zur Analyse der Zusammensetzung von SAV.
   Bahn 17-23: Proben von Überständen mock-transfizierter 293T CD5L::CD3::scFv::CD14 #19 Zellen
   Bahn 26-32: Proben von Überständen von 293T CD3::scFv::CD14 #19 Zellen nach Cotransfektion mit CD80, CD54, DC-SIGN und OGP.
   Antikörper:
   Bahn 17, 26: anti gag p30
   Bahn 18, 27: anti CD14
   Bahn 19, 28: anti flu-Tag
   Bahn 20, 29: anti CD54
   Bahn 21, 30: anti CD99
   Bahn 22, 31: anti CD59
   Bahn 23, 32: anti HLA Klasse I
**Abb. 3a:** Schema eines ELISA mit SAV zum Nachweis von Molekülen auf den SAV.
**Abb. 3b:** Ergebnis eines ELISA mit SAV nach Abb 3a; Messung der OD(405nm) zu den angegebenen Zeitpunkten nach Zugabe von p-Nitrophenylphosphat als Substrat für die alkalische Phosphatase.
**Abb. 4:** Proliferation von PBMNCs nach Stimulation mit SAV von unterschiedlich transfizierten Zellen, dargestellt in counts per minute (cpm) nach dreitägiger Vorkultur und anschließendem 18-stündigen methyl-3H-Thymidin-Einbau.

Im nachfolgenden wird die Erfindung anhand von Beispielen weiter erläutert. Die Erläuterungen dienen ausschliesslich der besseren Verständlichmachung der Erfindung und erlauben keinerlei Rückschlüsse auf eine Einschränkung der Erfindung auf die angeführten Beispiele.

### Beispiel 1: Herstellung der subzellulären antigenpräsentierenden Vesikel (SAV)

Ein Verfahren zur Herstellung der erfindungsgemässen SAV umfasst die folgenden Schritte:

### a) Konstruktion von CD5L::CD3-scFᵥ::CD14

Als Ausgangsmaterial wurde eine Hybridomzelle verwendet, welche den anti-human CD3 Antikörper OKT3 sezerniert (American Type Culture Collection). Mittels der dem Fachmann bekannten "salting out procedure" wurde genomische DNA aus dieser Zelllinie gewonnen, welche als Basis für die Herstellung von synthetischen DNA-Abschnitten aus den hypervariablen Regionen der rearrangierten Immunglobulingene VH und VL dienten. Zu diesem Zwecke wurden die entsprechenden Genabschnitte mit folgenden Primern amplifiziert (Amplifikationsbedingungen waren: 25 Zyklen 94 C 10 sek, 55 C 10 sek, 72 C 15 sek) :

VH: for GGAATTCGCTAGCCCAGGTCCAGCTGCAGCAGTCT,
rev GGGGGATCCGGTGACCGTGGTGCCTTGGCCCCAGTA;

VL: for GGAATTCGAGCTCCCAAATTGTTCTCACCCAGTCTCCA,
rev GGGATCCCCACCGCCCCGGTTTATTTCCAACTTTGTCCC.

Die Primer wurden dabei so gewählt, dass VH in seinem 5' Ende eine Nhe I, im 3' Ende eine Bst EII, VL in seinem 5'-Ende eine Sac I, in seinem 3' Ende eine Bam HI Restriktionsschnittstelle hatte. Nach entsprechender Restriktionsbehandlung wurden die VH bzw. VL Genabschnitte mit den durch Restriktionsendonuklease (RE) modifizierten Enden entsprechend verdaut und in die mit RE behandelten pBlueskript II KS+ Vektoren eingebracht und der Nucleotidsequenzanalyse zugeführt. Entsprechende Klone, welche die vorhergesagten Nukleotidsequenzen aufwiesen, wurden sodann über die sie flankierenden RE-Schnittstellen freigesetzt und gemeinsam mit einer Sac I und Bst EII flankierten Linkersequenz, welche für die Aminosäuren Gly und Ser in der Sequenz (G₃S)₄ kodiert, in den Vektor pBlueskript CD5LnegI eingebracht. Die erhaltene Sequenz wird nachfolgend als CD5L::CD3-scFᵥ ::neg1 bezeichnet. pBlueskript CD5LnegI enthält im 5' Ende eine vom CD5 Gen stammende Signalsequenz sowie die humanen Immunoglobulin G₁ Sequenzen welche für die Scharnierregion (hinge) sowie die konstanten Abschnitte CH2 und CH3 kodieren. An der Übergangsstelle zwischen Signalsequenz und Scharnierregion befinden sich die RE-Schnittstellen BamHI und Nhe I welche wie oben beschrieben für die Insertion des single chain Antikörpers verwendet wurden. Wird unter der Kontrolle eines eukaryontischen Promoter exprimiert, wie in unserem Fall in dem Expressionvektor pEAK12 und somit nachfolgend als pEAK12 CD5L::CD3-scFᵥ::IgG₁ bezeichnet, kommt es zur Produktion eines löslichen humanen IgG1 Antikörpers mit anti-human CD3 Spezifität (*Abb. 1a*).

Um eine membranständige, in Lipidrafts "getargetete" Expression des single chain Antikörpers zu erreichen, wurden die hinge-CH2 und CH3-Sequenzen durch die Ektodomäne der CD14 Sequenz, welche mittels synthetischer Oligonukleotide und unter Einbringung von BamHI und Not I Schnittstellen unter Zuhilfenahme der PCR Technologie modifiziert wurde, ersetzt und somit der Vektor pEAK12 CD5L::CD3-scFᵥ::CD14 hergestellt.

### b) Expression von pEAK12 CD5L::CD3-scFᵥ::CD14 und pEAK12 CD5L::CD3-scFᵥ::IgG₁.

293T Zellen wurden in der nach Wurm et al. (Jordan et al., 1996) modifizierten Form der Kalziumphosphatmethode mit 20 µg DNA per 4.5 10⁶ Zellen in einer 10 cm Petrischale transfiziert. Typischerweise wurde dazu eine Transfektionsmischung von 2 ml hergestellt und auf die Zellen, welche sich typischerweise in 15 ml IMDM Medium mit 10 % fötalem Kälberserum befinden, aufgebracht. 16 Stunden später wurde das Medium zur Gänze gewechselt und die Zellen für zwei (pEAK12 CD5L::CD3-scFᵥ::CD14) bzw. 9 (pEAK12 CD5L::CD3-scFᵥ::IgG₁) weitere Tage kultiviert.

Stabile Expression wurde durch Einbringung eines mit Avr II linearisierten pEAK12 CD5L::CD3-scFᵥ::CD14 Vektors und anschliessender Selektion in 1µg/ml Puromycin erreicht. 20 stabile Transfektanten wurden kloniert, für die weiteren Experimente wurde Klon 19 verwendet. Seine Expression von CD5L::CD3-scFᵥ::CD14 wurde mittels einer sogenannten Oberflächenfärbung von transfizierten 293T Zellen mittels anti-CD14 monoklonalen Antikörpern (mAk) oder anti-maus Ig Reagenzien, welche mit einem Fluorochrom markiert waren, durchgeführt (*Abb. 1d*). Die Funktionstüchtigkeit des single chain Antikörpers wurde durch die Herstellung des löslichen CD5L::CD3-scFᵥ::IgG₁. Moleküls und durch dessen exklusive Bindung an humane T-Zellen erfolgreich nachgewiesen.

### c) Konstruktion von kostimulatorischen, Adhäsions- und HLA Molekülen mit einer GPI-Verankerungssequenz im Expressionsvektor pEAK12

Die Konstruktion von CD5L::CD80::CD16 wurde ausgehend von den entsprechenden cDNAs, welche für die entsprechenden Proteinabschnitte kodieren, unter Zuhilfenahme von durch PCR Technologie modifizierten Enden der einzelnen Sequenzabschnitte durchgeführt. Die hergestellte Expressionskassette machte sich dabei die RE-Schnittstellen Hind III und Bam HI (für CD5L), Bam HI und Nhe I (für das entsprechend zu exprimierende Gen) sowie Nhe I und Not I (für die GPI Verankerungssequenz) zunutze (*Abb. 1b*).
Beispielhaft seien die Sequenzen der Oligonukleotide, welche für die Herstellung einiger für Ektodomänen kodierender Molekülabschnitte verwendet wurden, angeführt:

CD80 for: CGCGGGGATCCCGTTATCCACGTGACCAAGGA;

CD80 rev: GCGCCGCTAGCGGATGGGAGCAGGTTATCAG;

CD86 for: CGCGGGGATCCCGCTCCTCTGAAGATTCAAGC;

CD86 rev: GCGCCCGCTAGCGTGGTCTGGGGGAGGCTGAG;

CD40 for: CGCGGGGATCCCGAACCACCCACTGCATGCAG;

CD40 rev: GCGCCCGCTAGCCACCAGGGCTCTCAGCCGAT;

Die CD16-GPI Verankerungssequenz wurde mit den folgenden Oligonukleotiden hergestellt: CD16 for: CGCGGGGCTAGCTTGGCAGTGTCAACCATCTCA; CD16 rev: GCGCCCGCGGCGCTTTAAATGTTTCTCTTCACAGAGA; Jene für die CD59-GPI Verankerungssequenzen wie folgt:

CD59 for: GGATTCGCTAGCCTTGAAAATGGTGGGACATCC;

CD59 rev: CCCGCGGCGGCCGCTTTAGGGATGAAGGCTCCAGGCTG.

HLA-Klasse I Gene wurden unter Zuhilfenahme folgender Oligonukleotide modifiziert und wie in Abb. 1b in die Expressionskassette eingefügt: Beispielhaft seien die Oligonukleotidsequenzen für das HLA-A*02011 Gen angegeben:

HLA-A*02011 for: CGCGGGGATCCCTGACCCAGACCTGGGCG;

HLA-A*02011 rev: CGCGGGGATCCCCTGACCCAGACCTGGGCA;

Als Basis für die Herstellung der cDNA Abschnitte dienten die im Labor von Brian Seed in Boston, Mass., USA, klonierten cDNAs für die entsprechenden Moleküle oder aber cDNA Banken von PHA Blasten, der Raji oder Jurkat Zellinie, oder aber von Plazenta Gewebe.

### d) pEAK12.OG bzw. pEAK12.OGP Konstrukt (Abb. 1c)

Als Basis für die Konstruktion der GAG bzw. GAG-POL exprimierenden Plasmide wurde pEAK12.OGP herangezogen, welches unter Verwendung der pΨ- Sequenz als Asc I/Not I Fragment hergestellt wurde (Pickl et al., 2001). Um die *enν* Genabschnitte zu entfernen wurde die Sca I RE-Schnittstelle, welche sich am Beginn des MoMLV *enν* Genes befindet sowie die synthetisch hergestellte Not I RE Schnittstelle am Ende von *enν* verwendet. Nach Auffüllung des durch Not I-Behandlung entstandenen Überhanges mithilfe des Klenow Fragmentes von DNA-Polymerase I und Durchführung einer sogenannten blunt-end Ligation wurde pEAK12.OGP rezirkularisiert. Die Herstellung von pEAK12.OG wurde unter Zuhilfenahme des *gag*-spezifischen Primers CGCGGGGCGGCCGCTTTAGTCATCTAGGGTCAGGAGGG und eines pEAK12 Vorwärtsprimers mit der Sequenz CGACTCACTATAGGGAGAC amplifiziert, nachfolgend mit den RE Hind III und Not I verdaut und in den pEAK12 Expressionsvektor inseriert.

### e) Herstellung von exprimierbaren sogenannten Peptidminigenen (Anderson et al., 1991).

Tumor- und Viruspeptide bekannter HLA Restriktion werden als Minigene am 3' Ende des hocheffizienten CD5 Leaders (Signalsequenz) in pEAK12 kloniert. Dabei verwenden wir zur Steigerung der Expression in humanen Zellen codonoptimierte, synthetische Oligonukleotidpaare, welche Bam HI sowie Not I kompatible Enden besitzen und somit in den CD5L tragenden Vektor ligierbar sind.

### f) Transfektion von Produkten aus c) und d) in 293T CD5L::CD3-scFᵥ::CD14 stabile Transfektanten, Ernten der Überstände

In einem Vorversuch zur Funktion der erfindungsgemäßen SAV wurde das Erstsignal der APZ durch Expression des CD5L::CD3-scFᵥ::CD14 Fragments bereitgestellt. Die Transfektion der wie unter c) beschriebenen kostimulatorischen Moleküle und Adhäsionsmoleküle wurde mit oben genannter Kalziumphosphatmethode nach Wurm durchgeführt. Ebenfalls transfiziert wurde das unter d) beschriebene OGP-Konstrukt zu einem bei allen Ansätzen konstanten Anteil an der gesamten tranfizierten DNA von 25%. 16 Stunden nach der Transfektion wurde das Medium zur Gänze gewechselt und die Zellen wurden für zwei weitere Tage kultiviert. Die Überstände wurden geerntet (15 ml pro Ansatz) und durch Zentrifugation (670g in einem SORVALL RTH-250 Rotor, 10 min, Raumtemperatur) und anschließende Filtration durch einen 0,45 um Filter von zellulären Überresten befreit.
Im Sinne der Erfindung wurde in der Folge das CD5L::CD3-scFᵥ::CD14 Fragment ersetzt: Das Erstsignal besteht nunmehr in einem HLA Klasse I Molekül (komplexiert mit β₂Mikroglobulin) und einem von ihm präsentierten immunogenen Peptid. Dazu wird die Mutterzelllinie 293T mit dem entsprechenden HLA-Molekül und dem β₂Mikroglobulin sowie dem zu präsentierenden Peptid (zu gleichen Teilen), geeigneten Adhäsions- und kostimulatorischen Molekülen und dem OGP-Konstrukt transfiziert. Die die erfindungsgemäßen SAV enthaltenden Überstände werden in derselben Weise gewonnen und gereinigt.

Im folgenden werden morphologische und funktionelle Analyse sowie Anwendung der SAV anhand von weiteren Beispielen beschrieben.

### Beispiel 2: Morphologische Analyse

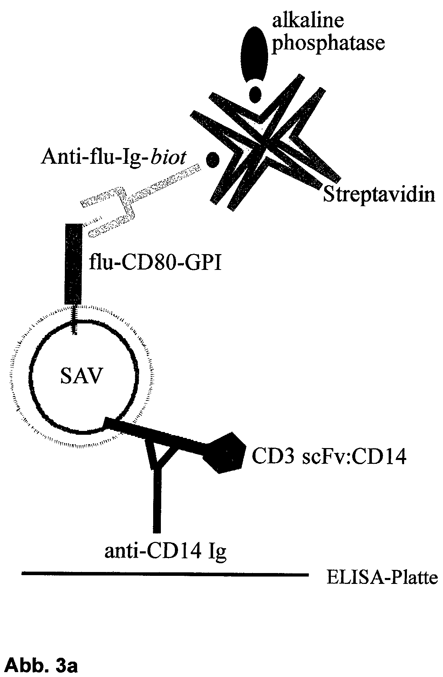

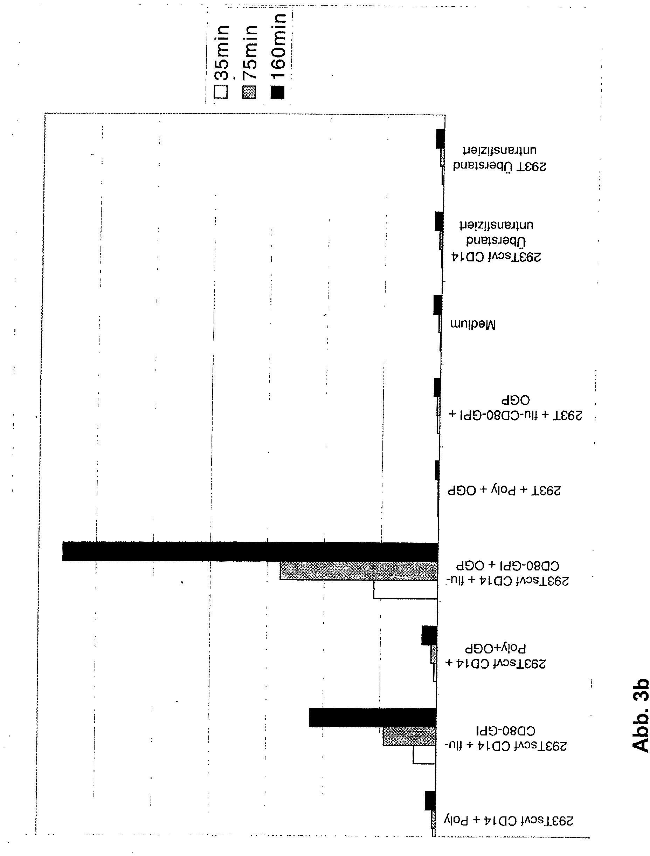

Die die immunogenen Vesikel enthaltenden Überstände wurden einer Ultrazentrifugation (100 000g in einem Beckman SW-60 Rotor, 1h, 4°C) unterzogen; anschliessend wurde das Pellet unter denselben Zentrifugationsbedingungen einmal mit PBS gewaschen, um dann in üblicher Weise zum Laden auf ein Polyacrylamid-Gel präpariert zu werden. Im Western Blot konnten in den Proben die transfizierten Moleküle hoch angereichert nachgewiesen werden (*Abb. 2*) ebenso wie zellinieneigene Lipidraft-spezifische Moleküle, was belegt, dass die immunogenen Überstände pelletierbare Elemente, subzelluläre Vesikel, enthalten und dass diese die gezielt in den Lipidrafts konzentrierten Moleküle enthalten.
Im ELISA konnte zusätzlich gezeigt werden, dass sich die unterschiedlichen transfizierten Moleküle im Überstand auf ein und derselben Struktur (d.h. SAV) befinden *(Abb 3).* Die Überstände können ohne Verlust der Partikelintegrität und ihrer biologischen Aktivität bis zu 4 Tage lang bei 4°C oder bei 37°C aufbewahrt werden.

### Beispiel 3: Funktionelle Analyse

In Proliferationstests wurde die Wirkung der die subzellulären Vesikel enthaltenden Überstände auf periphere mononukleäre Zellen aus menschlichem Blut (PBMNCs) untersucht. Dazu wurden in 96-well-Platten 100.000 Zellen/well/100µl mit Überständen (100µl/well) in einer speziellen,bei Virusinfektionen vielfach bewährten, Technik der "Spin-Inoculation" 2h bei 670g in einem SORVALL RTH-250 Rotor bei 30°C zentrifugiert und anschliessend 3 Tage lang im CO₂ Inkubator inkubiert. Danach wurde die Proliferation der PBMNCs über 18h anhand des Einbaus von ³H-Thymidin gemessen. (*Abb. 4*)
In Zytotoxizitätstests wurde die Aktivierung von zytotoxischen T-Zellen durch die subzelluläre Vesikel enthaltenden Überstände untersucht. Dabei wurden PBMNCs mit Überständen 7 Tage stimuliert. Anschließend wurden in Abständen von ca. 10 Tagen die PBMNCs restimuliert, und zwar mit Zytokinen (vorzugsweise IL-2, IL-7 und/oder IL-12) bzw. mit frischen Überständen von neu transfizierten Zellen.
Zur Auswertung wurden HLA-typisierte Zielzellen mit ⁵¹Chrom markiert, in gestaffelten Verhältnissen mit den stimulierten PBMNCs gemischt und 4h inkubiert. Die Überstände dieser Reaktion wurden geerntet, und im Gammazähler wurde deren Gehalt an Radioaktivität gemessen.

### Beispiel 4: Herstellung und Anwendung einer Tumorvakzine

Erfindungsgemäß sind die hierin beschriebenen SAV für die Anwendung am Menschen konzipiert. Sie werden dazu vorzugsweise mit den oben beschriebenen Adhäsions-, kostimulatorischen und patienten-spezifischen (nach vorangehender Ermittlung des Gewebeverträglichkeitstypus des Patienten) HLA-Molekülen sowie kompatiblen Antigen-Peptiden ausgestattet, um die gewünschte Aktivierung oder Inhibierung von Effektorzellen zu erreichen. Zu diesem Zweck werden SAV von 293T Zellen in serumfreiem Medium oder in humanserum-hältigem Medium produziert.
Anschließend werden die SAV durch Ultrazentrifugation (100.000g/lh/4°C) konzentriert, in PBS gewaschen und schließlich resuspendiert. Alternativ dazu kann dem Konzentrationsschritt ein Aufreinigungsschritt in einem Sucrose-Gradienten vorangehen.
Eine Konzentrationsbestimmung der SAV-hältigen Lösung wird mit dem oben beschriebenen ELISA unter Zuhilfenahme von anti-CD55 bzw. anti-CD59 monoklonalen Antikörpern, als Fängersubstanz auf die ELISA-Platten aufgebracht, durchgeführt. Betroffenen Krebspatienten wird eine effektive Dosis der SAV-hältigen Lösung injiziert, vorzugsweise intradermal, oder in Form eines Nasensprays nasal appliziert.

### Beispiel 5: Herstellung und Anwendung von anergisierenden SAV

Anergisierende SAV werden wie in Beispiel 4 beschrieben hergestellt und verabreicht, mit dem Unterschied, dass anergisierende SAV patientenspezifische HLA-Moleküle (nach vorheriger Ermittlung des Gewebeverträglichkeitstypes des Patienten) sowie kompatible Allergene oder Autoantigene als gegebenenfalls alleinige Komponente exprimieren.
Alternativ werden solche Kombinationen von kostimulatorischen und Adhäsionsmolekülen auf den anergisierenden SAV koexprimiert, welche gemeinsam mit den patienten-spezifischen HLA-Molekülen sowie den kompatiblen Allergenen oder Autoantigenen zur antigen-spezifischen Unempfänglichkeit von Effektorzellen führen. Gegebenenfalls werden auf anergisierenden SAV patientenspezifische HLA-Moleküle sowie kompatible Allergene oder Autoantigene mit solchen Kombinationen von kostimulatorischen und/oder Adhäsionsmolekülen koexprimiert, welche Suppressorzellen des Immunsystems aktivieren.

### Referenzen

Anderson, K., Cresswell, P., Gammon, M., Hermes, J., Williamson, A., and Zweerink, H. (1991). Endogenously synthesized peptide with an endoplasmic reticulum signal sequence sensitizes antigen processing mutant cells to class I- restricted cell-mediated lysis, J Exp Med *174*, 489-92.

Camacho, S. A., Heath, W. R., Carbone, F. R., Sarvetnick, N., LeBon, A., Karlsson, L., Peterson, P. A., and Webb, S. R. (2001). A key role for ICAM-1 in generating effector cells mediating inflammatory responses, Nat Immunol *2,* 523-9.

Johnstone, R. M., and Ahn, J. (1990). A common mechanism may be involved in the selective loss of plasma membrane functions during reticulocyte maturation, Biomed Biochim Acta *49,* S70-5.

Jordan, M., Schallhorn, A., and Wurm, F. M. (1996). Transfecting mammalian cells: optimization of critical parameters affecting calcium-phosphate precipitate formation, Nucleic Acids Res *24,* 596-601.

Latouche, J. B., and Sadelain, M. (2000). Induction of human cytotoxic T lymphocytes by artificial antigen- presenting cells, Nat Biotechnol *18,* 405-9.

Marschang, P., Sodroski, J., Wurzner, R., and Dierich, M. P. (1995). Decay-accelerating factor (CD55) protects human immunodeficiency virus type 1 from inactivation by human complement, Eur J Immunol 25, 285-90.

Melkonian, K. A., Ostermeyer, A. G., Chen, J. Z., Roth, M. G., and Brown, D. A. (1999). Role of lipid modifications in targeting proteins to detergent- resistant membrane rafts. Many raft proteins are acylated, while few are prenylated, J Biol Chem *274,* 3910-7. Pessin, J. E., and Glaser, M. (1980). Budding of Rous sarcoma virus and vesicular stomatitis virus from localized lipid regions in the plasma membrane of chicken embryo fibroblasts, J Biol Chem *255,* 9044-50.

Pickl, W. F., Pimentel-Muinos, F. X., and Seed, B. (2001). Lipid rafts and pseudotyping, J Virol 75, 7175-83.

Saifuddin, M., Ghassemi, M., Patki, C., Parker, C. J., and Spear, G. T. (1994). Host cell components affect the sensitivity of HIV type 1 to complement- mediated virolysis, AIDS Res Hum Retroviruses *10,* 829-37.

Sprent, J., Cai, Z., Brunmark, A., Jackson, M. R., and Peterson, P. A. (1997). Constructing artificial antigen-presenting cells from Drosophila cells, Adv Exp Med Biol *417*, 249-54.

Thery, C., Boussac, M., Veron, P., Ricciardi-Castagnoli, P., Raposo, G., Garin, J., and Amigorena, S. (2001). Proteomic analysis of dendritic cell-derived exosomes: a secreted subcellular compartment distinct from apoptotic vesides, J Immunol *166,* 7309-18.

Zitvogel, L., Regnault, A., Lozier, A., Wolfers, J., Flament, C., Tenza, D., Ricciardi-Castagnoli, P., Raposo, G., and Amigorena, S. (1998). Eradication of established murine tumors using a novel cell-free vaccine: dendritic cell-derived exosomes, Nat Med 4, 594-600.

## Patentansprüche

1. Antigen präsentierendes, vorzugsweise subzelluläres, Vesikel, **dadurch gekennzeichnet, dass** es an seiner Oberfläche entweder relevante Moleküle für Erst- und vorzugsweise Zweitsignale zur antigenspezifischen Aktivierung von T-Lymphozyten oder relevante Moleküle für Erstsignale zur antigenspezifischen Anergisiserung bzw. zur Induktion von negativ-regulatorischen T-Lymphozyten enthält.

2. Vesikel nach Anspruch 1, **dadurch gekennzeichnet, dass** es als Molekül für ein Erstsignal zur Aktivierung von T-Lymphozyten ein anti-CD3-Fragment und/oder ein HLA-Molekül samt einem daran gebundenen immunogenen Peptid, vorzugsweise einem tumorassoziierten oder viralen Antigen, enthält.

3. Vesikel nach Anspruch 1 oder 2, **dadurch gekennzeichnet, dass** es als Molekül für ein Zweitsignal zur Aktivierung von T-Lymphozyten mindestens ein kostimulatorisches Molekül und/oder mindestens ein Adhäsionsmolekül enthält.

4. Vesikel nach Anspruch 3, **dadurch gekennzeichnet, dass** es mindestens ein kostimulatorisches Molekül aus der Gruppe CD80, CD86 und CD58 enthält.

5. Vesikel nach Anspruch 3 oder 4, **dadurch gekennzeichnet, dass** es mindestens ein Adhäsionsmolekül aus der Gruppe CD54 (ICAM-1) und DC-SIGN (CD209) enthält.

6. Vesikel nach Anspruch 1, **dadurch gekennzeichnet, dass** es als Molekül für ein Erstsignal zur Anergisiserung bzw. zur Induktion von negativ-regulatorischen T-Lymphozyten mindestens ein HLA-gebundenes Allergen oder Autoantigen und gegebenenfalls zusätzlich mindestens einen Rezeptor enthält, der ein inhibitorisches Signal an Effektor T-Lymphozyten vermittelt.

7. Vesikel nach einem der Ansprüche 1 bis 6, **dadurch gekennzeichnet, dass** mindestens ein Molekül für ein Erst- oder Zweitsignal und/oder mindestens ein Molekül aus der Gruppe kostimulatorische Moleküle und Adhäsionsmoleküle ein Typ I Transmembranprotein ist und an seinem 3'-Ende eine Lipidraft-targeting Sequenz besitzt.

8. Verfahren zur Herstellung von antigenpräsentierenden, vorzugsweise subzellulären, Vesikeln nach Anspruch 1, umfassend die folgenden Schritte:
a) Klonierung von relevanten Molekülen für Erst- und gegebenenfalls Zweitsignale zur antigenspezifischen Aktivierung oder Anergisierung von T-Lymphozyten, insbesondere aus der Gruppe der modifizierten HLA-Moleküle, Antigenpeptide, kostimulatorischen Molekülen und Adhäsionsmoleküle, sowie eines Coreproteins oder Coreprotein-Fragments eines Hüllenvirus, vorzugsweise des gag-pol- bzw. des gag Fragments von Moloney MLV, in Expressionsvektoren;
b) Transfektion mindestens eines Expressionsvektor-Konstrukts zur antigenspezifischen Aktivierung oder zur antigenspezifischen Anergisierung von T-Lymphozyten sowie mindestens eines rekombinanten viralen Coreproteins oder Coreprotein-Fragments in ein eukaryotisches Zellkultursystem und Expression der Konstrukte durch Inkubation des Zellkultursystems, wobei antigenpräsentierende Vesikel von den Zellen des Zellkultursystems abgeschnürt werden; und
c) Ernte und Reinigung der antigenpräsentierenden Vesikel aus dem Überstand der transfizierten Zellkultur nach der Inkubation.

9. Verfahren nach Anspruch 8, worin als Zellkultursystem die Zellline 293 T eingesetzt wird.

10. Verfahren nach Anspruch 8 oder 9, worin als virales Coreproteinfragment das GAG Protein von Moloney MLV eingesetzt wird.

11. Verfahren nach einem der Ansprüche 8 bis 10, worin die rekombinant eingebrachten Moleküle für Erst- und gegebenenfalls Zweitsignale sowie jene für die Coreproteine überexprimiert werden.

12. Verfahren nach einem der Ansprüche 8 bis 11, unter Verwendung einer Expressionskassette für ein CD5L::CD3-scFᵥ::CD14 Fragment, insbesondere unter Verwendung der Expressionskassette gemäss Fig. 1a.

13. Verfahren nach einem der Ansprüche 8 bis 11, unter Verwendung einer Expressionskassette für modifizierte Ektodomänen von Transmembranproteinen des Typs I, insbesondere unter Verwendung einer Expressionskassette gemäss Fig. 1b.

14. Verfahren nach einem der Ansprüche 8 bis 13, unter Verwendung einer Expressionskassette für das gag-pol- bzw. gag-Fragment des Moloney MLV gemäss Fig. 1c.

15. Vesikel nach Anspruch 1, erhältlich in einem Verfahren gemäss einem der Ansprüche 8 bis 14.

16. Vakzine, **dadurch gekennzeichnet, dass** sie subzelluläre Vesikel nach einem der Ansprüche 1 bis 7 und 8 enthält.

17. Vakzine nach Anspruch 16, **dadurch gekennzeichnet, dass** die enthaltenen HLAund Peptidmoleküle spezifisch auf einen Empfänger abgestimmt sind.

18. Vakzine nach Anspruch 16 oder 17, **dadurch gekennzeichnet, dass** die enthaltenen HLA- und Peptidmoleküle spezifisch auf ein virales oder tumorassoziiertes Antigen, gegen das T-Lymphozyten aktiviert werden sollen, abgestimmt sind.

19. Vakzine nach Anspruch 16 oder 17, **dadurch gekennzeichnet, dass** die enthaltenen HLA- und Peptidmoleküle spezifisch auf ein Allergen oder Autoantigen, gegen das Anergie erzeugt werden soll, abgestimmt sind.
